# EUROPÄISCHE PATENTSCHRIFT

(11) **EP 3 318 532 B1**
(45) Veröffentlichungstag und Bekanntmachung des Hinweises auf die Patenterteilung: **25.06.2025**
(21) Anmeldenummer: 16196843.3
(22) Anmeldetag: 02.11.2016
(51) Int. Cl.: C01B 21/24, A61K 33/00

(54) **VERFAHREN UND EINRICHTUNG ZUR NO INSTILLATION**
METHOD AND DEVICE FOR NO INSTILLATION
PROCÉDÉ ET DISPOSITIF D'INSTILLATION DE NO

(43) Veröffentlichungstag der Anmeldung: 09.05.2018
(73) Patentinhaber: BSN medical GmbH, 22761 Hamburg (DE)
(72) Erfinder: Schulze, Christian, 22337 Hamburg (DE); Schütz, Patrick, 12159 Berlin (DE)
(74) Vertreter: Cohausz & Florack

(56) Entgegenhaltungen:
- US-A1- 2013 028 942
- US-A1- 2015 157 657
- US-A1- 2016 051 579

## Beschreibung

### Gegenstand der Erfindung

Die vorliegende Erfindung betrifft ein mehrstufiges Dilutionsverfahren zur Herstellung einer Stickstoffmonoxid-haltigen Lösung. Die Erfindung betrifft weiterhin eine Vorrichtung zur Durchführung des erfindungsgemäßen Dilutionsverfahrens und eine Instillationseinheit umfassend diese Vorrichtung. Die Erfindung betrifft zudem die Vorrichtung und die Instillationseinheit in ihrer Verwendung zur Behandlung von Erkrankungen, insbesondere von diabetisch bedingten Durchblutungsstörungen und chronischen Wunden.

### Hintergrund der Erfindung

Aus dem Stand der Technik sind zahlreiche Verfahren und Vorrichtungen zur Herstellung von Stickstoffmonoxid (NO) bekannt.

Gemäß der EP 1 903 003 A1 kann NO durch Photolyse einer photolabilen NO-Vorstufe hergestellt werden, wobei die die Reaktion unter Anwesenheit von Radikalfängern und Antioxidantien zur Bildung von hochreinem NO verläuft. Bei diesem Verfahren ist in der Anwendung auf die NO-Erzeugung innerhalb von Flüssigkeiten in der Regel nur mit einem langsamen Anfluten der NO-Konzentration zu rechnen.

Gemäß der WO2013/063354 kann ein NO-freisetzendes Fußbad hergestellt werden, indem ein Polysiloxan-Polymer, das mit Diazeniumdiolatgruppen derivatisiert ist der Badlösung zugegeben wird. Dieses reagiert dann mit Wasser unter Bildung von NO. Da die NO-Generierung hierbei durch einen spontanen Zerfall der Polymerseitengruppen erfolgt, kann die Freisetzungskinetik hierbei nur unzureichend gesteuert werden. Zudem dauert es bei diesem Verfahren eine geraume Zeitspanne, bis ein therapeutisch relevanter NO-Spiegel aufgebaut ist.

Die US 2016/051579 A1 betrifft ein Verfahren zur Behandlung von Rinder-Atemwegserkrankungen durch Stickstoffmonoxid und lehrt im Beispiel 2 ([0280] und [0281]) das Ansäuern einer Natriumnitritlösung mit Citrat als Feststoff, um eine NO-freisetzende Lösung herzustellen. 900 µl der erhaltenen NO-freisetzenden Lösung werden mit 100 µl einer Bakterien-Präparation gemischt und die erhaltene Lösung anschließend seriell verdünnt.

Die US 2013/0028942 A1 betrifft die Herstellung von NO-und Nitrit-haltigen Aerosolen, auf Basis einer Lösung, die vorab mit Zitronensäure angesäuert wird (Beispiel 1, [0381]).

Die US 2015/0157657 A1 betrifft die Herstellung und Bereitstellung von Lösungen mit nachhaltiger NO-Freisetzung. In der US'657 wird eine wässrige Lösung aus NaNO₂ vorgelegt, mit fester Zitronensäure angesäuert und schnell vermischt.

Es besteht daher noch Bedarf an neuen Verfahren zur Herstellung NO-haltiger Lösungen, in denen schnell und dennoch in kontrollierter Weise NO in hoher Reinheit erzeugt werden kann.

Aufgabe der Erfindung ist es daher ein Verfahren zur Herstellung von therapeutisch einsetzbaren NO-Lösungen bereitzustellen, das bezüglich mindestens einer der oben genannten Nachteile verbessert ist.

### Zusammenfassung der Erfindung

Diese Aufgabe wird gemäß der Erfindung dadurch gelöst, dass ein Verfahren zur Herstellung einer Stickstoffmonoxid (NO)-haltigen Lösung bereitgestellt wird, das die folgenden Schritte umfasst:
(a) Bereitstellen einer ersten Reaktionskomponente umfassend einen pH-labilen NO-Donor;
(b) Bereitstellen einer zweiten Reaktionskomponente, die eine wässrige Lösung mit einem pH-Wert zwischen 2,5 und 7,0 ist;
(c) Vermischen der ersten und zweiten Reaktionskomponente unter Bildung einer wässrigen Lösung mit einem pH-Wert zwischen 3,0 und 5,5 und Generierung von NO zur Ausbildung einer NO-haltigen wässrigen Lösung;
(d) Verdünnen der NO-haltigen wässrigen Lösung aus Schritt (c) durch Vermischen mit einer wässrigen Verdünnungslösung, so dass ein Verdünnungsfaktor von 5 bis 20 resultiert, und die wässrige Verdünnungslösung eine gepufferte Lösung oder destilliertes Wasser ist, so dass der pH-Wert der Mischung aus Schritt (c) um mindestens eine pH-Wertstufe erhöht wird.

Spezifische Ausgestaltungen der Erfindung sind Gegenstand weiterer abhängiger und unabhängiger Ansprüche.

Das erfindungsgemäße Verfahren vereinigt mehrere entscheidende Vorteile gegenüber den aus dem Stand der Technik bekannten Verfahren.

Es zeigte sich in überraschender Weise, dass dieses Verfahren den komplementären Anforderungen einer NO-Freisetzungskinetik gerecht wird. So kann im aziden Milieu sehr schnell eine geeignete, konzentrierte Stammlösung an NO in der wässrigen Lösung aufgebaut werden, die dann über den Vorgang des Vermischens mit einer wässrigen Verdünnungslösung eine pH-Werterhöhung erfährt. Dadurch kann die NO-Konzentration über einen längeren Zeitraum stabilisiert werden und so in kontrollierter Weise aufrechterhalten werden.

Über die getrennte Vorhaltung der ersten und zweiten Reaktionskomponente kann eine zeitlich exakt steuerbare und damit bedarfsgerechte Bereitstellung der NO-haltigen Stammlösung erfolgen.

Üblicherweise erschwert die kurze Halbwertszeit des NO den therapeutischen Einsatz. Mit dem erfindungsgemäßen Verfahren kann trotz der kurzen Halbwertszeit durch eine Stabilisierung des NOs in dem schwach sauren, neutralen oder basischen Trägermedium der NO-Spiegel für eine ausreichende Zeitspanne aufrechterhalten werden und gleichzeitig eine therapeutisch relevante Konzentration eingestellt werden.

Durch die Anwesenheit von Antioxidantien erlaubt das Verfahren die Herstellung von NO in einer Reinheit, wie sie für die therapeutische oder kosmetische Anwendung erforderlich ist.

Aus dem Stand der Technik sind zahlreiche pH-labile NO-Donoren bekannt, wie beispielsweise Nitritsalze, NONOate oder Nitrosothiole, auf die der Fachmann hier zurückgreifen kann.

Aufgrund der hochkontrollierten Steuerung der Freisetzung kann das Verfahren in Vorrichtungen eingesetzt werden, die das NO nur in sehr geringen Mengen freisetzen. Dies ist insbesondere bei NO als hochpotentem bioaktivem Molekül ein entscheidender Vorteil. Zudem erlaubt dies die Entwicklung einer entsprechenden Vorrichtung (wie bspw. eine Wundauflage oder ein Fußbad) als Medizinprodukt (z.B. als sog. Medical device class III), insofern in diesen speziellen Fällen eine Vorrichtung vorliegt, bei der die Wirkung primär durch die mechanischen oder physikalischen Eigenschaften der Vorrichtung bedingt ist.

Durch einfache Anpassung des Verfahrens bezüglich NO-Donoren und Säuren kann es gezielt an die Behandlungserfordernisse angepasst werden.

Durch das erfindungsgemäße Verfahren kann auch auf eine externe Zuführung von NO verzichtet werden.

Bei dem erfindungsgemäßen Verfahren handelt es sich um ein einfaches Verfahren mit größtenteils bekannten Substanzen, so dass es sowohl schnell und kostengünstig durchzuführen ist, als auch bei geringer Fehleranfälligkeit einfach in der therapeutischen Anwendung ist.

Das mehrstufige Dilutionsverfahren ist durch das separate Bereithalten von Reaktionskomponenten, die in dieser Form stabil und ungiftig sind, für eine Automatisierung des Herstellungsverfahrens besonders gut geeignet.

Aufgrund der vorabgenannten Vorteile eignet sich das Verfahren insbesondere für einen Einsatz in der Unterdruckwundtherapie, so dass hier die Wunde mit einer NO-haltigen Spüllösung gespült werden kann und dann nach Absaugen der Lösung einem therapeutisch vorteilhaften Unterdruck ausgesetzt werden kann.

Die mit dem Herstellungsverfahren betriebenen Vorrichtungen eröffnen im Hinblick auf die kennzeichnenden Parameter und auf die Materialauswahl weitere Freiheitsräume.

Zusammenfassend ermöglicht das erfindungsgemäße NO-Herstellungsverfahren NObasierte Therapieformen, bei denen preiswert, zuverlässig, sicher und für den Anwender individualisierbar das hochreaktive, und entsprechend instabile Gas NO in kontrollierter Weise applizierbar ist.

### Die Erfindung im Einzelnen

Die Erfindung beinhaltet somit ein mehrstufiges Verfahren, bei dem zunächst über das Vermischen der ersten und der zweiten Reaktionskomponente eine NO-Generierung im sauren Milieu induziert wird und nach einer ausgewählten Zeitdauer der pH-Wert anschließend über eine Verdünnung dieses Gemischs mit einer wässrigen Verdünnungslösung erhöht wird, um die pH-abhängige NO-Neusynthese zu stoppen oder zu verringern und eine NO-haltige wässrige Lösung mit einer therapeutisch anwendbaren NO-Konzentration bereitzustellen.

Durch die pH-Werterhöhung in den bevorzugterweise leicht sauren, neutralen oder basischen Bereich unterbleibt die Neugenerierung von toxischen NO₂-Radikalen. Durch die erfindungsgemäße Anwesenheit des mindestens einen Antioxidans werden NO₂-Radikale und andere bei der NO-Generierung entstehenden Radikale eliminiert, so dass die wässrige Flüssigkeit mit hochreinem NO angereichert ist.

Ausgangspunkt des Verfahrens ist die erste Reaktionskomponente, die einen pH-labilen Donor umfasst und in bevorzugter Wiese auch ein Antioxidans enthält.

Die NO-Generierung wird durch Vermischung dieser ersten Reaktionskomponente mit der zweiten Reaktionskomponente gestartet. Die zweite Reaktionskomponente ist hierbei eine wässrige Lösung mit einem pH-Wert zwischen 2,5 und 7,0. Durch die Zugabe der ersten Reaktionskomponente, welche den pH-labilen NO-Donor umfasst, wird durch Bereitstellung oder Herstellung eines aziden Milieus die NO-Generierung gestartet. Beachtlicherweise kann die zweite Reaktionskomponente auch einen schwach sauren oder neutralen pH-Wert aufweisen, der als solches nicht geeignet ist, die NO-Herstellung zu induzieren. Allerdings kann die erste Reaktionskomponente neben dem pH-labilen Donor auch eine sauer reagierende Substanz (wie bspw. Zitronensäure) aufweisen, die nach der Vermischung mit der wässrigen zweiten Reaktionskomponente einen entsprechend sauren pH-Wert ergibt.

Für kosmetische oder medizinische Anwendungen muss das Gemisch weiterhin zu dem Zeitpunkt, an dem ein azider pH-Wert die NO-Generierung erlaubt, zusätzlich mindestens ein Antioxidans umfassen. Hierzu kann das mindestens eine Antioxidans bereits in der ersten Reaktionskomponente mit dem pH-labilen NO-Donor vorliegen und mit diesem zur zweiten Reaktionskomponente zugegeben werden. Im Folgenden werden NO-Donor und Antioxidans auch als Substanzklassen bezeichnet.

In einer Ausführungsform ist die erste Reaktionskomponente ein Gemisch umfassend einen pH-labilen NO-Donor und mindestens ein Antioxidans. In einer bevorzugten Ausführungsform liegen bei der ersten Reaktionskomponente der pH-labile Donor und das mindestens eine Antioxidans in von einander getrennten Kompartimenten vor, so dass sie erst nach gemeinsamer Vermischung mit der zweiten Reaktionskomponente in einem Kompartiment vorliegen. In einer besonders bevorzugten Ausführungsform liegen bei der ersten Reaktionskomponente das Nitrit als pH-labiler NO-Donor und die Ascorbinsäure oder ein Salz davon (also ein Ascorbat) in getrennten Kompartimenten vor.

Die getrennte Kompartimente können durch ein Gefäß oder Bereich mit einer Trennwand oder Trennmembran gebildet werden. Alternativ kann eine Substanzklasse oder sogar beide Substanzklassen, also NO-Donor und Antioxidans, in verkapselter Form (z.B. als Mikrokapsel) vorliegen, so dass die Kapselwand eine Trennbarriere darstellt. Auch bei einer Mischung der beiden verkapselten Substanzklassen oder der verkapselten mit der nichtverkapselten Substanzklasse würden sie somit in voneinander getrennten Kompartimenten vorliegen.

Alternativ kann das mindestens eine Antioxidans auch in der wässrigen, sauren Lösung der zweiten Reaktionskomponente vorliegen. Dies hat den Vorteil, dass die in dem Trägermedium enthaltenen Bestandteile auch schon während der Produktion und/oder Lagerung durch das anwesende, mindestens eine Antioxidans vor unerwünschter Oxidation geschützt sind. Dies kann gerade bei verfahrensgemäßen Vorrichtungen wie Wundauflagen oder Pflastern von Vorteil sein, da hier die Zugabe von weiteren Substanzen schwer möglich ist und diese eine ausreichende Lagerstabilität aufweisen müssen.

Die erste Reaktionskomponente umfasst erfindungsgemäß als essentiellen Bestandteil einen pH-labilen NO-Donor, also eine Substanz, die in der Lage ist, in azidem wässrigem Milieu NO zu erzeugen. Bevorzugt liegt diese erste Reaktionskomponente als Feststoff, Schaum, Gel, Creme oder Flüssigkeit vor. In besonders bevorzugter Weise liegt die erste Reaktionskomponente als Feststoff vor, der besonders bevorzugt ein pulverförmiger oder granulärer Feststoff ist.

Erfindungsgemäß wird als zweite Reaktionskomponente eine wässrige Lösung mit einem pH-Wert von 2,5 bis 7,0 verwendet. Durch Mischung mit der ersten Reaktionskomponente entsteht hierbei eine wässrige Lösung mit einem pH-Wert, der in einem Bereich von 3,0 bis 5,5, bevorzugt in einem Bereich von 3,5 bis 5,3 und besonders bevorzugt in einem Bereich von 4,0 bis 5,0 liegt.

Erfindungsgemäß wird zur Verdünnung der wässrigen NO-haltigen Lösung eine wässrige Verdünnungslösung verwendet. Hierbei kann jede wässrige Lösung verwendet werden, die physiologisch unbedenklich ist und in der das NO in der therapeutisch relevanten Konzentration löslich ist. Erfindungsgemäß ist die Verdünnungslösung eine gepufferte Lösung oder destilliertes Wasser.

Erfindungsgemäß wird für die NO-Generierung eine pH-labile NO-Vorstufe (auch NO-Donor (NOD) genannt) eingesetzt. pH-labile NO-Donoren sind im Stand der Technik bekannt und dem Fachmann geläufig.

In einer bevorzugten Ausführungsform der Erfindung sind die pH-labilen NO-Donoren ausgewählt aus der Gruppe enthaltend organische Nitrate, anorganische Nitrate, anorganische Nitrite, organische Nitritester wie Alkylnitrite, Schwefel-, Stickstoff- oder Sauerstoff-Nitrosoverbindungen, NO-Metall-Verbindungen und NO-chelatierende Substanzen.

Beispiele für pH-labile NOD umfassen anorganische Nitrite, Alkylnitrite wie Isopentylnitrit, Diazeniumdiolat-Derivate, trans[RuCl([15]aneN4)NO]²⁺, 6-Nitrobenzo[a]pyrol, S-Nitroso-Glutathion, S-Nitroso-Thiol, S-Nitroso-N-Acetyl-D-Penicillamin (SNAP), Nitroanilin-Derivate, 2-Methyl-2-Nitrosopropan, Imidazoyl-Derivate, Nitratester, Hydroxylnitrosamin, Hydroxylamin, Hydroxyharnstoff und Natrium-Nitroprussid.

In bevorzugter Weise ist der pH-labile NO-Donor ein anorganisches Nitritsalz, das zweckmäßigerweise eine pharmakologisch verträgliche Substanz darstellt. Als solche kommen beispielsweise Nitrite von Alkali- oder Erdalkalimetallen zur Anwendung. Beispielhaft sind hier genannt: LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, oder Ra(NO₂)₂ und Kombinationen hiervon.

Besonders bevorzugt ist hierbei als NOD das NaNO₂, das in weiterhin bevorzugter Weise zusammen mit einer Kombination aus Ascorbinsäure und/oder Trolox als Antioxidantien in der ersten Reaktionskomponente oder in dem Reaktionsgemisch eingesetzt werden.

Die Konzentration des Nitritsalzes oder der Nitritsalze in der wässrigen Lösung als Gemisch aus erster und zweiter Reaktionskomponente beträgt bevorzugt zwischen 1 und 100 mM, besonders bevorzugt zwischen 5 und 70 mM und insbesondere zwischen 10 und 50 mM.

In einer alternativen Ausgestaltung kann auch ein Nitratsalz verwendet werden, bei dem eine enzymatische Umwandlung in das entsprechende Nitritsalz möglich ist. Bevorzugt sind hierbei Nitrate von Alkali- oder Erdalkalimetallen zur Anwendung. Beispielhaft sind hier genannt: LiNO₃, NaNO₃, KNO₃, RbNO₃, CsNO₃, FrNO₃, Be(NO₂)₃, Mg(NO₂)₃, Ca(NO₂)₃, Sr(NO₂)₃, Ba(NO₂)₃, oder Ra(NO₂)₃.

Um die bei der NO-Generierung auftretenden mehrfach oxidierten Stickoxide, Sauerstoffradikalanionen oder Hydroxylradikale zu entfernen, ist es für eine kosmetische oder medizinische Anwendung notwendig, dass bei der Reaktion mindestens ein Antioxidans anwesend ist. Daher enthält bei dem erfindungsgemäßen Verfahren die erste Reaktionskomponente und/oder die zweite Reaktionskomponente und/oder die Verdünnungslösung mindestens ein Antioxidans.

In bevorzugter Weise enthält nur die erste Reaktionskomponente das mindestens eine Antioxidans. Dieses mindestens eine Antioxidans wird dann zusammen mit dem pH-labilen NO-Donor in der wässrigen Lösung als zweite Reaktionskomponente gelöst und kann so direkt zu Beginn der NO-Generierung seine antioxidative Wirkung entfalten.

Nach Art des chemischen Wirkmechanismus werden Antioxidantien in Radikalfänger oder Reduktionsmittel unterschieden.

Bei Oxidationsreaktionen zwischen organischen Verbindungen treten vielfach kettenartige Radikalübertragungen auf. Hier werden Stoffe mit sterisch behinderten Phenolgruppen wirksam, die im Ablauf dieser Übertragungen reaktionsträge, stabile Radikale bilden, die nicht weiter reagieren, wodurch es zum Abbruch der Reaktionskaskade kommt (Radikalfänger). Zu ihnen zählen natürliche Stoffe wie die Tocopherole und synthetische wie Butylhydroxyanisol (BHA), Butylhydroxytoluol (BHT) und die Gallate.

Des Weiteren können auch Reduktionsmittel mit einem sehr niedrigen Standard-RedoxPotential von weniger als +0,4 V (bei pH 7,0 und 25°C) eingesetzt werden. Typische Vertreter sind etwa Ascorbinsäure (-0,04 V bei pH 7 und 25°C), Salze der Schwefligen Säure (+0,12 V bei pH 7 und 25 °C) und bestimmte organische schwefelhaltige Verbindungen (z. B. Glutathion, Cystein, Thiomilchsäure).

In einer bevorzugten Ausführungsform ist das mindestens eine Antioxidans in der Lage das als NO-Donor im sauren Milieu vorliegende HNO₂ zu NO zu reduzieren. Hierzu muss das Antioxidans als Reduktionsmittel ein Standard-Redoxpotential von weniger als +1,0362 Volt, bevorzugt von weniger als +0,5 Volt, besonders bevorzugt von weniger als +0,2 Volt und insbesondere bevorzugt von weniger als 0 Volt aufweisen.

Das mindestens eine Antioxidans ist zweckmäßigerweise in der Lage das schädliche NO₂-Radikal zum NO₂-Anion zu reduzieren. Für eine effektive Eliminierung des NO₂ Radikals sollte das mindestens eine Antioxidans bevorzugt eine bimolekulare Reaktionskonstante k aufweisen, die größer als 1,0 x 10⁶ M⁻¹s⁻¹ und bevorzugt größer als 1,0 x 10⁷ M⁻¹s⁻¹ ist. Erfindungsgemäß geeignete Antioxidantien mit den dazugehörigen Reaktionskonstanten sind in Kirsch et al., 2002 (Biol. Chem 383; 389 -399, s. Tabelle 1) offenbart. Beispielhaft seien hier genannt: Captoprilthiolat, Kaffeesäure, Sinapinsäure, Ferulasäure, Lycopen, Zeaxanthin, Lutein, Astaxanthin, Canthaxanthin, Arachidonat, Gly-Tyr-Dipeptid, Tyrosin, Purine und Pyrimidine wie die Nucleobasen Adenin, Guanin, Cytosin, Thymin, Uracil und die entsprechenden Derivate und Analoga hiervon inklusive der sie enthaltenden Nucleoside und Nucleotide.

In einer weiteren Ausführungsform umfasst die erfindungsgemäß verwendete wässrige Lösung neben dem Antioxidans auch einen Antioxidationssynergisten. Synergisten unterstützen die Wirkung von Antioxidantien, indem sie beispielsweise verbrauchte Antioxidantien wieder regenerieren (sog. "Redox cycling"). Durch Komplexierung von Metallspuren (Natrium-EDTA) oder Schaffung eines oxidationshemmenden pH-Wertes können Synergisten die antioxidative Wirkung eines Radikalfängers oder Reduktionsmittels verstärken. Typische Beispiele für Antioxidationssynergisten sind EDTA, 1-Hydroxyethan-1.1-diphosphonsäure, Citronensäure, Fumarsäure, Harnsäure und 2-(Hydroxymethyl)-1,4-benzyldiol.

Bei dem erfindungsgemäßen Herstellungsverfahren wird besonders bevorzugt das Ascorbat oder die Ascorbinsäure als Antioxidans eingesetzt.

Für die erfindungsgemäßen wässrigen Lösungen eignen sich besonders wasserlösliche Vitamin E-Derivate wie Trolox oder alpha-AMG, organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und Phytoöstrogene.

Für die wässrige Reaktionslösung können zweckmäßigerweise wasserlösliche Vertreter der vorabgenannten Gruppen kombiniert werden, also beispielsweise Ascorbat und (RS)-6-Hydroxy-2,5,7,8-tetramethylchroman-2-carbonsäure (Trolox), Ascorbat und Cystein, oder in bevorzugter Weise Ascorbat und N-Acetylcystein.

Zweckmäßigerweise liegt das mindestens eine Antioxidans im Verhältnis zu dem NO-Donor in einem molaren Überschuss vor.

In einer Ausführungsform der Erfindung enthält die zweite Reaktionskomponente in ihrer Ausgestaltung als wässrige Flüssigkeit zusätzlich einen oder mehrere der folgenden Stoffe: Katalysatoren, Detergentien, Puffersubstanzen, Chromophore, Substanzen, die das Prodrug stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung der Reaktionskomponente zur topischen Anwendung physiologische verträgliche und/oder dermatologisch verträgliche Stoffe und Stoffgemische zur Anwendung kommen.

### Säureaktivierung im Schritt (c)

Für die Spaltung des pH-labilen NO-Donors wird die erste Reaktionskomponente mit der zweiten Reaktionskomponente gemischt, so dass ein ausreichend saurer pH-Wert resultiert. Dieser pH-Wert liegt hierbei erfindungsgemäß so niedrig, dass er die Spaltung des pH-labilen NO-Donors unter Bildung von NO induziert. Der konkrete pH-Wert hängt von der pH-Labilität des NO-Donors und der erwünschten Zeitspanne für die NO-Generierung ab. Je geringer der pH-Wert, desto schneller wird im Reaktionsgemisch das NO erzeugt werden. Dieses Reaktionsgemisch dient nach Generierung des NOs als NO-haltige Stammlösung für den nachfolgenden Verdünnungsschritt (Schritt d).

Gemäß der Erfindung beträgt der pH-Wert im Schritt (c) hierbei zwischen 3,0 und 5,5 bevorzugt zwischen 3,5 und 5,2, besonders bevorzugt zwischen 4,0 und 5,0 und insbesondere bei 5,0. Der optimale Wert für den pH-Wert ist wie oben bereits ausgeführt von dem jeweils verwendeten NO-Donor und der intendierten Reaktionsgeschwindigkeit abhängig und kann vom Fachmann entsprechend eingestellt werden.

In bevorzugter Weise besitzt der Verfahrensschritt der NO-Generierung, gemessen ab der Durchmischung der ersten und zweiten Reaktionskomponente eine Zeitdauer von zwischen einer Minute und 60 Minuten, bevorzugt zwischen 15 Minuten und 45 Minuten und besonders bevorzugt von 20 bis 30 Minuten.

Die durch die Reaktion entstandene, NO-haltige, wässrige Stammlösung wird mit einer wässrigen Verdünnungslösung um einen Faktor zwischen 5 und 20, bevorzugt zwischen 8 und 12 und besonders bevorzugt um den Faktor 10 so verdünnt, dass der pH-Wert der NO-haltigen Stammlösung um mindestens eine pH-Wertstufe erhöht wird.

Zweckmäßigerweise weist die durch den Verdünnungsschritt (d) entstandene Lösung eine NO-Konzentration zwischen 10 µM und 1000 µM, bevorzugt von zwischen 20 µM und 500 µM und besonders bevorzugt von zwischen 75 µM und 200 µM auf.

### Pharmakologische aktive Substanzen

In einer Ausführungsform der Erfindung enthält eine der Reaktionskomponenten zusätzlich eine oder mehrere pharmakologisch aktive Substanzen. Diese können die pharmakologische Wirkung des NOs unterstützen oder unabhängig von dem NO in einer für die entsprechende Erkrankung therapeutisch relevanten Weise wirken.

Als pharmakologisch aktive Substanzen seien hier beispielhaft genannt: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

### Vorrichtung

Es ist eine weitere Aufgabe der vorliegenden Erfindung, eine verbesserte Vorrichtung zur Herstellung einer therapeutisch einsetzbaren NO-haltigen wässrigen Lösung zur Verfügung zu stellen.

In einem weiteren Aspekt stellt die Erfindung eine Vorrichtung zur Durchführung des erfindungsgemäßen Verfahrens bereit, das mindestens drei Behälter wie folgt umfasst:
(a) ein erster Behälter zur Aufnahme der ersten Reaktionskomponente, der über eine Öffnung oder Leitung mit einem zweiten Behälter verbunden ist,
(b) ein zweiter Behälter zur Aufnahme der flüssigen zweiten Reaktionskomponente, wobei dieser Behälter darüber hinaus mit einer weiteren Öffnung oder Leitung zum Durchlass von Flüssigkeit mit einem dritten Behälter verbunden ist; und
(c) ein dritter Behälter zur Aufnahme der Verdünnungsflüssigkeit,
wobei die Leitung oder Öffnung zwischen den einzelnen Behältern durch ein Absperrelement verschlossen, dass sich öffnen oder entfernen lässt, um die Verbindung zwischen den einzelnen Behältern herzustellen, und wobei der erste Behälter ein mit der ersten Reaktionskomponente vorbefüllter Verschlussdeckel ist, der den zweiten Behälter abschließt und einen Auslösungsmechanismus aufweist, der bei Betätigung eine Vermischung der ersten Reaktionskomponente im Verschlussdeckel und der zweiten Reaktionskomponente im zweiten Behälter erlaubt.

In einer bevorzugten Weise ist der Auslösungsmechanismus ein Druckknopf oder der Deckel ist als drehbarer Deckel mit Gewinde ausgestaltet. Bei dem drehbaren Deckel kann ein an der Unterseite des Deckels angebrachtes Durchstechelement (z.B. ein dornförmiges oder ein klingenförmiges Element) bei der Drehung und der durch das Gewinde resultierenden Abwärtsbewegung des Verschlussdeckels die Trennmembran durchstechen und damit die Mischung der beiden Reaktionskomponenten ermöglichen. Entsprechend kann der Druckknopf bei dem Eindrücken durch ein an der Unterseite des Deckels angebrachtes Durchstechelement die Trennmembran durchstechen und damit zur Vermischung führen. Zweckmäßigerweise besteht der erste Behälter, der bevorzugt ein vorbefüllter Verschlussdeckel ist, ebenso wie auch die Trennwand aus einem chemisch inerten Material.

In besonders bevorzugter Form umfasst der Verschlussdeckel wie oder mehrere Kompartimente, in denen einzelnen Substanzklassen wie pH-labiler NO-Donor und Antioxidans voneinander getrennt untergebracht werden können.

In einer weiteren Ausführungsform ist die Vorrichtung und hier der zweite Behälter mit einer Mischvorrichtung versehen, die für eine schnelle und vollständige Durchmischung der Reaktionskomponenten mit der wässrigen Lösung sorgt. In einer bevorzugten Ausführungsform arbeitet diese Mischvorrichtung im geschlossen System der Vorrichtung, ohne dass ein direkter Eingriff in die Vorrichtung und hierbei in die Behälter notwendig ist, so sollte z.B. von außen kein Rührstab eingeführt werden müssen. Dies kann beispielsweise durch ein magnetisch arbeitendes Rührelement erreicht werden. Hierbei kann ein magnetisches Rührstäbchen (sog. "Rührfisch") verwendet werden, der durch eine benachbart aber außerhalb der Vorrichtung vorliegende magnetische Rührvorrichtung angetrieben wird, wobei diese ein rotierendes magnetische Feld erzeugt.

Ein weiteres Beispiel stellt eine Aufnahme für Behälter 1 und 2 dar, die z.B. Vibrationen auf diesen überträgt, um so ein Lösen der Reaktionskomponenten zu erreichen.

In einer bevorzugten Ausführungsform wird daher diese Mischvorrichtung durch eine außerhalb der Vorrichtung gelegene Rührvorrichtung, die bevorzugt Teil der Installationseinheit ist, in kontrollierter Weise gesteuert und/oder angetrieben.

In einer bevorzugten Ausführungsform ist der zweite Behälter und/oder der dritte Behälter als plastisch verformbarer Kunststoffbeutel ausgestaltet. Dies ermöglicht weitere Strategien zur einfachen und "nicht-invasiven "Durchmischung der Rektionskomponenten in der wässrigen Lösung.

Dem Fachmann stehen hierzu zahlreiche Kunststoffmaterialen zur Verfügung, wobei diese bevorzugt ausgewählt sind aus der Gruppe enthalten Polypropylen, Polyethylen, Ethylenvinylacetat (EVA), Polyvinylchlorid (PVC), Polyethylenterephthalat (PET), orientiertes Polypropylen (OPP), biaxial orientiertes Polypropylen (BOPP), orientiertes Polyamid (OPA) und biaxial orientiertes Polyamid (BOPA).

In einer weiteren Ausführungsform besteht der Beutel aus mehrschichtiger Folie auf Polyolefinbasis. Beispielhaft seien hier genannt: Polyethylen / Ethylvinylalkohol-Copolymer / Polyethylen (PE/EVOH/PE), PP/EVOH/PP, PP/EVOH/PE, Polyamid/Polyethylen (PA/PE), PE/PA/PE, PP/PA/PE, PA/EVOH/PA/PE and PP/PA/EVOH/PA/PE, EVOH/OPP, EVOH/BOPP, EVOH/OPA, EVOH/BOPA und PVDC/PET.

So kann die die den Beutel aufnehmende Einheit, also bevorzugt die Instillationseinheit, einen Trägerplatte umfassen, auf die der Beutel aufgelegt wird, wobei die Trägerplatte bewegbar ist und so ausgestaltet ist, dass sie eine horizontale Rotations-, eine Wipp-, eine Vibrations- oder eine Schüttelbewegung ausführen kann.

In einer alternativen Ausführungsform umfasst die den Beutel aufnehmende Einheit eine Druckvorrichtung, die auf den Beutel einen Druck ausüben kann. Dieser Druck wird bevorzugt nur auf einen Teilbereich des Beutels ausgeübt, so dass die Flüssigkeit in benachbarte Bereiche des Beutels gedrückt wird und nach Nachlassen oder Aufheben des Druckvorgangs durch Zurückverteilen der Flüssigkeit die Durchmischung erfolgt.

Dem Fachmann stehen zahlreiche Möglichkeiten für die Ausgestaltung einer solchen Druckvorrichtung bereit. Beispielhaft sein hier genannt:
- Die Druckvorrichtung kann als Stempel ausgestaltet sein, der den bevorzugterweise flachliegenden Beutel seitlich zusammendrückt und durch alternierendes Abheben und Absenken des Stempels zur Durchmischung des Beutelinhalts führt.
- Die Druckvorrichtung kann eine Rolle darstellen, die den Beutel partiell eindrückt und durch eine Hin-und Herbewegung den Beutelinhalt vermischt.
- Die Druckvorrichtung kann ein Exzenterelement sein, das über eine asymmetrisch angeordnete Drehachse einen periodisch auf den Beutel ausgeübten Druck erzeugt.

In einer weiteren Ausführungsform umfasst die Vorrichtung und/oder die die Vorrichtung aufnehmende Einheit eine Transportiervorrichtung zum Transport der Flüssigkeit aus dem zweiten Behälter in den dritten Behälter. Beispielhaft seien hier die folgenden Transportiervorrichtungen genannt, die einzeln oder in Kombination eingesetzt werden können.

In einer ersten Ausführungsform steht der dritte Behälter unter Unterdruck, so das beim Öffnen des zwischen und dem zweiten und dritten Behälter angebrachten Ventils die Flüssigkeit in den dritten Behälter gezogen wird. Dies kann in einfacher Weise durch einen dritten Behälter erzielt werden der unter einer Formspannung steht, beispielweise durch einen Kunststoffbehälter aus einem Duromer, der über sein Füllvolumen hinaus entleert wurde, so dass er eine "Einbeulung" aufweist.

In einer zweiten Ausführungsform wird in dem zweiten Behälter ein Überdruck aufgebaut, der die Flüssigkeit in den dritten Behälter drückt. Dies kann beispielsweise endogen durch eine chemische Reaktion geschehen (z.B. durch Zugabe einer Carbonatsalzes in der ersten Reaktionskomponente, die im sauren Milieu der Flüssigkeit des zweiten Behälters zur CO₂-Bildung führt). Alternativ kann der zweite Behälter auch einen Gasanschluß aufweisen, durch das von außen Gas in den zweiten Behälter eingeführt wird und damit die Flüssigkeit in den dritten Behälter gedrückt wird. Hier gilt zur Lage des Anschlusses und zur Lage der Behälter das oben für die erste Ausführungsform Gesagte.

In einer dritten Ausführungsform fungiert die erfindungsgemäße Mischvorrichtung auch als Transportiervorrichtung. So könnten beispielweise die auf den Beutel einwirkende Rolle, bei Öffnung des Ventils zwischen diesen Behältern und einer zum dritten Behälter ausgeführten, den Beutel ausdrückenden Bewegung die Flüssigkeit in den dritten Beutel überführt werden.

In einer vierten Ausführungsform weist die zwischen dem zweiten und dritten Behälter vorgesehene Öffnung oder Leitung eine Anschlussmöglichkeit für eine Pumpvorrichtung auf. So kann bei einer Schlauchleitung die Flüssigkeit durch eine Schlauchpumpe, die bevorzugt als Peristaltikpumpe ausgestaltet ist, von dem zweiten Behälter in den dritten Behälter gepumpt werden.

In einer bevorzugten fünften Ausführungsform weist der erste Behälter an seinem oberen Ende, d.h. an dem Ende, das der Anschlussstelle zum dritten Behälter entgegengesetzt ist, einen Entlüftungsanschluss auf, so dass beim Öffnen diese Anschlusses und vertikaler Lagerung des zweiten Beutel oberhalb des dritten Beutels Luft in den zweiten Beutel eindringen kann und die Flüssigkeit nach unten in den dritten Beutel abfließen kann.

Es ist eine weitere Aufgabe der vorliegenden Erfindung, eine verbesserte Instillationseinheit zur Abgabe einer therapeutisch einsetzbaren NO-haltigen wässrigen Lösung zur Verfügung zu stellen.

In einem weiteren Aspekt stellt die Erfindung eine Instillationseinheit bereit, welche die erfindungsgemäße Vorrichtung umfasst und ausgewählt ist aus der Gruppe aufweisend Wundauflage, Wundpflaster, Spray, Inhalator, Badevorrichtung und Duschvorrichtung.

In einer Ausführungsform ist die Instillationseinheit so aufgebaut, dass sie die drei Behälter der Vorrichtung als integralen, d.h. fest montierten Bestandteil umfasst.

In einer alternativen Ausführungsform ist die Instillationseinheit so ausgestaltet, dass sie eine reversible Kopplung oder Aufnahme zu einem oder mehreren Behältern ermöglicht.

Die Instillationseinheit kann beispielsweise einen Hohlraum aufweisen, der die drei Behälter aufnimmt und den dritten Behälter mit der Instillationseinheit zur Abgabe der fertigen NO-haltigen Lösung verknüpft.

Bevorzugterweise umfasst die Instillationseinheit eine oder mehrere der folgenden Komponenten:
(a) Steuerungssystem zum Öffnen und Schließen der Absperrelemente zwischen den Vorrichtungsbehältern;
(b) ein Kontrollsystem zur Kontrolle des Öffnungsstatus der Absperrelemente;
(c) Pumpvorrichtung zum Transport oder Umwälzen der wässrigen Lösungen;
(d) ein Rührsystem zum Durchmischen der Flüssigkeiten in einem oder mehreren der Behälter der Vorrichtung;
(e) eine Temperiereinheit zur Temperierung der Reaktionskomponenten und oder der Verdünnungslösung und/oder der verdünnten NO-haltigen Lösung;
(f) eine Rechen- und Speichereinheit zur Steuerung der Komponenten der Instillationseinheit.

### Instillationseinheit mit Duschvorrichtung

In einer Ausführungsform umfasst die Instillationseinheit eine Duschvorrichtung.

Eine solche Duschvorrichtung verringert das Risiko der (Re-)kontamination von Wunden durch Mikroben von z.B. benachbarten Hautarealen, da die kontaminierte wässrige Lösung direkt von der Hautstelle abfließt und durch neue, nicht-kontaminierte wässrige Lösung ersetzt wird.

Im Gegensatz zu einem Eintauchbad wird die Haut nicht übermäßig aufgeweicht. Da zudem kein Eintauchbehälter gefüllt werden muss, ist diese Art der Anwendung auch schneller und die Behandlung kann direkt nach Generierung der wirkstoffhaltigen Badelösung beginnen.

Bei instabilen Wirkstoffen, wie beispielsweise NO, erlaubt die Duschanwendung in einfacherer Weise die Herstellung von Badelösungen mit konstanter NO-Konzentration.

Eine Duschvorrichtung erlaubt eine flexiblere Anwendung, wobei die Behandlung auf die notwendigen Körperbereiche fokussiert werden kann.

In einer Ausführungsform der Erfindung umfasst die Duschvorrichtung mehrere voneinander beabstandete Duschköpfe, die bevorzugt über eine gemeinsame Flüssigkeitsleitung miteinander verbunden sind.

In einer weiteren Ausgestaltung ist an dem Duschkopf eine Umstellvorrichtung vorgesehen, durch die mit dem Duschkopf verschiedene Strahlarten erzeugt werden können, beispielsweise ein normaler Wasserstrahl und ein Brausestrahl.

In einer besonderen Ausführungsform wird durch die Duschvorrichtung ein pulsierender Wasserstrahl erzeugt, der als Massagestrahl die therapeutische Wirkung gefäßerweiternder Wirkstoffe, wie beispielsweise NO, zusätzlich vorteilhaft unterstützt.

In einer besonderen Ausführungsform nutzt der Duschkopf das Prinzip der Venturidüse und ermöglicht die Vermischung einer wirkstofffreien Badelösung mit der wirkstoffhaltigen Badelösung. In bevorzugter Weise ist hierbei der in dem Duschkopf integrierten Behälter für die wirkstoffhaltige Badelösung mit der Wasserzufuhr verbunden. Durch die Verbindung mit der Venturidüse, die sich am Behälter befindet, wird die wirkstoffhaltige Badelösung aus dem Behälter transportiert und in dieser Mischung an die Duschkopflöcher weitergeleitet. Die Vermischung erfolgt beispielsweise durch Betätigung eines Schalters, der die Verbindung zwischen der Venturidüse und dem Wirkstoff-Badelösungsbehälter öffnet.

Die Duschvorrichtung ist zweckmäßigerweise so ausgestaltet, dass die Freisetzung des Wirkstoffs, insbesondere bei gasförmigen Wirkstoffen wie NO, aus der Badelösung in die Luft verhindert wird. Hierzu kann beispielsweise der Duschkopf eine randseitige Luftansaugung aufweisen, so dass das aus dem Wasserstrahlen austretende NO sofort abgesaugt wird und entweder im Duschkopf der Badelösung wieder zugeführt wird oder aus dem System (z.B. durch Filtration, Adsorption oder Abbau) entfernt wird.

Dies kann auch durch einen Duschkopf mit zwei unterschiedlichen Austrittsbereichen gewährleistet werden, wobei ein erster, innerer Bereich des Duschkopfs für die wirkstoffhaltige Badelösung vorgesehen ist und ein zweiter ringförmiger Austrittsbereich, der den inneren Bereich umschließt, für eine wirkstofffreie Badelösung vorgesehen ist. Dieser zweite Bereich bildet einen "Mantel" aus wirkstoff-freier Badelösung und sorgt dafür, dass der aus der Badelösung des ersten Bereichs austretende Wirkstoff hierein gelöst wird und nicht in die Umgebung gerät.

In einer weiteren Ausgestaltung der Erfindung ist die Duschvorrichtung nicht als Duschkopf gestaltet, sondern als Schlauch oder Rohr mit Austrittsöffnungen, wobei der Schlauch oder das Rohr bevorzugt als Ring oder als Spirale ausgeformt sind. In einer bevorzugten Ausführungsform ist der Ring oder die Spirale an der Innenwand einer Duschkammer der Duschvorrichtung angebracht und die Austrittslöcher weisen hierbei nach innen.

In einer bevorzugten Ausgestaltung, ist die Duschvorrichtung so ausgestaltet, dass sie auf dem zu behandelnden Körperteil aufgelegt oder befestigt werden kann und so in bevorzugter Weise einen Wasserfilm aufbaut, der an dem Körperteil abläuft. Diese Ausgestaltung hat den Vorteil, dass sie mit besonders geringen Mengen an wirkstoffhaltiger Badelösung auskommt und durch die Filmbildung (im Gegensatz zu einer Sprühvorrichtung) die Freisetzung von potenziell toxischen Wirkstoffen in die Umwelt besonders gut verhindert. Für diese Ausgestaltung kann der Schlauch oder der (Halb)ring partiell oder vollständig um das zu behandelnde Körperteil herumgeführt werden und beispielsweise durch eine leichte Klemmwirkung an diesem arretiert werden. In einer alternativen Ausgestaltung, kann die Duschvorrichtung auch als Bügel ausgestaltet sein, der in seiner Form an das zu behandelnde Körperteil angepasst ist.

In einer besonderen Ausführungsform ist an dem oben erwähnten Ring, Schlauch- oder Bügel ein Duschvorhang befestigt. Bei der körperseitigen Befestigung dieser Duschvorrichtungen verhindert dieser körpernahe Duschvorhang in zusätzlichem Maße die Freisetzung des Wirkstoffs.

In einer weiteren Ausgestaltung ist die Duschvorrichtung als Drainagestrumpf, Bandage oder Handschuh ausgestaltet und erlaubt so eine gezielte körperseitige Freisetzung des Wirkstoffs.

Des Weiteren kann die Duschvorrichtung mit einer oder mehreren Körperabdeckungen kombiniert werden, so dass nur der zu behandelnde Bereich für die Duschanwendung zugänglich ist. In einer bevorzugten Ausführungsform kann diese Abdeckung eine oder mehrere Aussparungen für den zu behandelnden Körperbereich aufweisen.

In einer weiteren Ausgestaltung ist bei der Duschvorrichtung die Austrittsöffnung schlitzförmig ausgestaltet, so dass die Duschvorrichtung als Schwalldusche fungiert. Gegenüber einem Duschkopf mit vielen einzelnen Wasserstrahlen, führt so eine Schwalldusche zu einer geringeren Freisetzung des Wirkstoffs in die Umgebung.

Zweckmäßigerweise ist die Duschvorrichtung mit einem Schalter ausgestattet, der die Wasserzufuhr regelt.

Darüber hinaus kann die Duschvorrichtung noch einen Druckregler aufweisen, der den Wasserdruck und damit die austretende Wassermenge reguliert.

### Instillationseinheit in Kombination mit der NPWT-Wundauflage

In einer bevorzugten Ausführungsform wird die Instillationseinheit in Verbindung mit einer Wundauflage verwendet. Hierzu kann die Installationseinheit mit einer Wundauflage derart gekoppelt werden, dass eine Spülung der Wunde mit der NO-haltigen Lösung ermöglicht wird. Die von der Vorrichtung gemäß dem erfindungsgemäßen Verfahren hergestellte NO-haltige Lösung wird durch eine Leitung zu der Wundauflage transportiert, um dort in den Raum zwischen Wunde und Wundauflage eingebracht zu werden und dann die Wunde zu spülen. Hierbei ist die Verwendung einer Peristaltikpumpe bevorzugt, da sie einen Pumpvorgang ohne Verunreinigung der Pumpe erlaubt.

Zweckmäßigerweise ist die Wundauflage mit einer Ansaugeinrichtung versehen, so dass nach dem Spülen der Wunde die Lösung wieder abgesaugt werden kann.

In bevorzugter Weise ist diese Ansaugeinrichtung so gestaltet, dass sie im Wundraum unterhalb der Wundauflage einen Unterdruck aufbaut, der besonders bevorzugt zwischen -60 und -200 mm Hg liegt. Insbesondere ist die Ansaugeinrichtung fähig einen Unterdruck von -60, -70, -80, -85, -90, -100, -110, -120, -130, -140, -150, -160, -170, -180, -190 oder - 200 mm Hg aufzubauen.

### Computerprogrammprodukt

In einem Aspekt offenbart die Anmeldung ein Computerprogrammprodukt, welches direkt in eine Speichereinheit geladen werden kann und Softwareabschnitte umfasst, mit denen das Verfahren gemäß einem der Ansprüche ausgeführt werden kann, wenn das Computerprogrammprodukt auf einer Installationseinheit ausgeführt wird.

### Therapeutische oder kosmetische Verwendung

In einem besonderen Aspekt stellt die Erfindung somit eine Vorrichtung zur Durchführung des erfindungsgemäßen Verfahrens bereit, die zur Verwendung bei der Behandlung oder Prävention von Erkrankungen geeignet ist, wobei der Patient dem aus der Vorrichtung freigesetzten NO ausgesetzt wird.

Bevorzugterweise erfolgt diese Behandlung mittels äußerlicher oder topischer Anwendung. So kann durch Auflegen eines NO-freisetzenden Pflasters oder einer Wundauflage auf einem therapiebedürftigen Hautareal dieses Areal gezielt behandelt werden.

Alternativ kann bei einer Badevorrichtung der erkrankte Körperteil durch Eintauchen in die NO-haltige Flüssigkeit behandelt werden oder aber durch Besprühen, Begießen oder Übergießen mit der NO-haltigen Flüssigkeit.

Die erfindungsgemäße Vorrichtung kann hierbei insbesondere zur Anregung des Stoffwechsels von Geweben durch äußerliche Anwendung, im Bereich der Dermatologie zur Behandlung von chirurgischen oder unfallbedingten Wunden, chronischen, nicht- bzw. schlechtheilenden und/oder bakteriell bzw. Pilz-befallenen Wunden sowie zur Behandlung von dermatologischen Erkrankungen aus dem Formenkreis der entzündlichen, immunologisch gesteuerten bzw. Autoimmunerkrankungen verwendet werden.

In einer bevorzugten Ausführungsform ist die mit der erfindungsgemäßen Vorrichtung behandelte Erkrankung ausgewählt aus der Gruppe enthaltend neuropathische Schmerzen, Krampfadern, Ischämien und thrombopathische Erkrankungen, Allergien, Hautinfektionen, Hautentzündungen, atopischen Dermatitis insbesondere Neurodermitis, Dermatomyositis und Pemphigus vulgaris; Wunddefekte, wie der chronischen diabetischneuropathischen Ulcus, Ulcus cruris, Dekubituswunden; primär heilende Wunden, sekundär heilende infizierte Wunden, Brandwunden, Hidradenitis supparativa (Akne inversa), Warzen, Windelausschlag, Rasurbrand, Komplikationen bei Hauttransplantationen, erektile Dysfunktion, Angina pectoris, Herzinsuffizienz, Linksherzinsuffizienz, Koronare Herzkrankheit, Pektanginöse Symptome nach Myokardinfarkt, Analfissur, Krämpfe der glatten Muskulatur des Oesophagus, Menstruationsbeschwerden, Reynaud-Syndrom, Buerger-Syndrom, periphere arterielle Erkrankung (PAD), periphere arterielle Verschlusskrankheit (pAVK), entzündliche und Autoimmunerkrankungen der Haut (Psoriasis, Dermatiden, Neurodermitis), Pilzerkrankungen der Haut, bakterielle, mikotische und parasitäre Erkrankungen der Haut (z.B. Leishmaniose), Tinea cruris und Tinea inguinalis.

Mit dem erfindungsgemäßen Verfahren hergestellte Lösungen können bevorzugt in Form eines Inhalationssprays zur Behandlung obstruktiver Lungenerkrankungen verwendet werden. Weiterhin können sie zur Induktion einer lokalen Vasodilatation von verengten oder verschlossenen Blutgefäßen eingesetzt werden. Hierbei ist es bevorzugt, die Lösung direkt in das Herz zu applizieren, beispielsweise durch eine endoskopische Maßnahme.

In einer Ausführungsform können mit der erfindungsgemäßen Vorrichtung lokale Durchblutungserkrankungen beim Tier, wie beispielsweise die Hufrehe beim Pferd behandelt werden und dabei generell veterinärmedizinische Erkrankungen, die den hier aufgelisteten Humanerkrankungen entsprechen oder nahekommen.

Die erfindungsgemäße Vorrichtung kann auch zur Behandlung einer Muskeldystrophie (MD) eingesetzt werden. Behandelbare MD-Formen umfassen hier: MD-Duchenne, MD-Becker-Kiener, Emery-Dreifuss_MD-Typ 1, Skapulopereonale MD, reducing body myopathy (RBM), Gliedergürteldystrophien, kongenitale Muskeldystrophien, distale Muskeldystrophien, "Vocal cord and pharyngeal weakness with distal myopathy" (VCPDM), Myofibrilläre Myopathien und Myotone Dystrophien.

Eine mit der erfindungsgemäßen Vorrichtung behandelbare Entzündung kann eine bakterielle, virale, mykotische oder parasitäre Infektion darstellen. Die bakterielle Infektion kann hierbei beispielsweise durch ein Bakterium verursacht werden, das ausgewählt ist aus der Gruppe enthaltend *S. aureus, B. circulans, B. cereus, E. coli,* P. vulgaris, P. *acnes, S. pyogenes, S. enterica, V. anguillarum, K. pneumoniae, P. piscicida, P. aeruginosa, A. tumefaciens, M tuberculosis,* und *M ulcerans.* Die Pilzinfektion kann durch einen Pilz hervorgerufen werden, der ausgewählt ist aus der Gruppe enthaltend T. *equinum, C. Albicans, F. oxysporum, R. solani, B. cinerea,* und *A. jlavus.* Bei der zu behandelnden Pilzinfektion kann die Haut oder Nagel gemäß einer Onychomycosis befallen sein. Virale Infektionen können durch eine der folgenden Virenfamilien hervorgerufen werden: Poxviridae, Rotaviren, Papillomaviren, Parvoviren, und Varicella-Viren. Bevorzugt kann die NO-freisetzende Vorrichtung zur Behandlung von Hautinfektionen eingesetzt werden, bei denen der Virus *Molluscum contagiosum* involviert ist. Die parasitäre Infektion kann beispielsweise durch einen Parasit der folgenden Gattungen entstehen: Plasmodium, Leishmania, Schistosoma, Austrobilharzia, Heterobilharzia, Ornithobilharzia oder Cryptosporidium. Hervorzuheben ist hier der Erreger *Plasmodium falciparum.*

In einer Ausführungsform kann die erfindungsgemäße Vorrichtung zur Behandlung der anfallsartigen bei der Sichelzellanämie auftretenden Durchblutungsstörungen (Sichelzellkrisen) verwendet werden. Für den in solchen Fällen eingesetzten Wirkstoff Hydroxyharnstoff wird vermutet, dass er bei den Erythrozyten die Ausbildung der deoxygenierten T-Variante hemmt, und so die Umwandlung in den Sichelzellphänotyp verhindert. Durch Anbindung des freigesetzten NOs an das Hämoglobin entsteht hingegen die nicht-sichelzellbildende R-Variante was mit einer Verbesserung der Durchblutung und sogar einer Unterbindung der Sichelzellkrisen einhergehen kann.

In einer weiteren Ausführungsform kann die erfindungsgemäße Vorrichtung zur Behandlung von Haarausfall und hierbei insbesondere der androgenetischen Alopezie eingesetzt werden. Die Behandlung schließt hier sowohl eine Verlangsamung oder einen Stopp des Haarausfalls mit ein und sogar das Neuwachstum von Haaren. Weitere Formen des Haarausfalls, die erfindungsgemäß behandelt werden können, umfassen Alopecia praematura, Alopecia areata, Alopecia areata atrophicans, Alopecia totalis, Alopecia universalis, diffuse Alopezie, Alopecia actinica, Alopecia mechanis wie Alopecia liminaris, Alopecia marginalis frontalis traumatica, Alopecia seborrhoica, Alopecia muciosa und Alopecia parvimaculata. Analog zur Wirkungsweise des Medikaments Minoxidil sollte das NO hierbei durch erhöhte Durchblutung der Kopfhaut eine verstärkte Versorgung der Haarfollikel mit Blut, Sauerstoff und Nährstoffen mit sich bringen.

Erfindungsgemäß kann die Vorrichtung beispielsweise wie folgt angewendet werden:
1.) auf offenen Wunden, da sich überraschenderweise herausgestellt hat, dass die erfindungsgemäße Anwendung nicht hautirritierend wirken;
2.) für die MRSA Prophylaxe in Risikopatienten; oder
3.) als synergistische Anwendung mit konventionellen Antibiotika, da sich überraschend herausgestellt hat, dass in Folge einer NO-Einwirkung, die konventionellen Antibiotika die verbleibende Entzündung effektiv zu bekämpfen vermag.

In einer bevorzugten Ausführungsform wird die erfindungsgemäße Vorrichtung zur Behandlung chronischer Wunden der unteren Extremitäten von Diabetikern verwendet. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

Zudem könnte es möglich sein, durch Behandlung größerer Körperareale auch systemische Erkrankungen, wie z.B. den erhöhten Blutdruck (Hypertonie) und verwandte hämodynamische Erkrankungen zu adressieren.

In einer Ausführungsform der Erfindung wird die erfindungsgemäße Vorrichtung zur Behandlung schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen sowie systemischen Wirkung dar, die das Risiko der Entstehung von Wunden signifikant mindert bzw. deren Heilung wesentlich beschleunigt. Das mittels der erfindungsgemäßen Vorrichtung dem zu behandelnden Körperteil zugeführte NO kann daher erfolgreich zur Therapie schlechtheilender Wunden angewendet werden.

In einer besonderen Ausführungsform wird die erfindungsgemäße Vorrichtung zur Behandlung des diabetischen Schmerz der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit der erfindungsgemäße Vorrichtung von außen dem Fuß und/oder Bein zugeführte NO kann daher erfolgreich zur Therapie des diabetischen Fuß-/Beinschmerzes angewendet werden.

In einer speziellen Ausführungsform der Erfindung wird die erfindungsgemäße Vorrichtung zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

In einer weiteren Ausführungsform stellt die Erfindung auch ein kosmetisches Verfahren bereit, bei dem das durch das erfindungsgemäße Verfahren oder die erfindungsgemäße Vorrichtung hergestellte NO auf die Haut des Menschen einwirkt.

### DEFINITIONEN

Im Rahmen der Erfindung ist eine "Reaktionskomponente" als eine Einzelsubstanz oder ein Gemisch von Substanzen definiert, die zur Reaktion der Vermischung mit einer anderen Reaktionskomponente bedürfen.

Erfindungsgemäß ist unter dem Begriff der "Behandlung" jegliche Anwendung der erfindungsgemäßen Vorrichtung am Individuum zu verstehen, die dazu dient, die Erkrankung symptomatisch oder kausal zu lindern oder sogar gänzlich zu unterdrücken oder das Voranschreiten der Erkrankung aufzuhalten, zu verzögern oder hinauszuschieben.

Gemäß der Erfindung ist unter "Instillation" die Verabreichung von flüssigen pharmazeutischen oder anderen medizinischen Zusammensetzungen auf Körperoberflächen oder in den Organismus zu therapeutischen, diagnostischen oder präventiven Zwecken zu verstehen. Eine Instillationseinheit ist dementsprechend eine Vorrichtung, die die Applikation der flüssigen pharmazeutischen Zusammensetzung durchführt.

Im Gegensatz zur Infusion, bei der die Medikamente durch den Kreislauf im gesamten Organismus verteilt werden (systemische Therapie), verbleibt das Medikament bei der Instillation am Ort der Applikation und wirkt dort lokal.

Im Kontext der vorliegenden Erfindung wird unter der "Prävention" die Vermeidung des Auftretens von Erkrankungen, und insbesondere von vaskulären oder Stoffwechsel-Erkrankungen verstanden und damit die Verringerung ihrer Verbreitung und die Verminderung ihrer Auswirkungen auf Morbidität und Mortalität der Bevölkerung. Die zentrale Strategie ist, die Auslösefaktoren von Krankheiten zurückzudrängen oder ganz auszuschalten.

Die Prävention umfasst hierbei sowohl primordiale Prävention, die Primärprävention, die Sekundärprävention, die Tertiärprävention und auch die Quartärprävention.

Primärprävention setzt vor Eintreten der Krankheit ein und zielt darauf ab, ein Neuauftreten einer Erkrankung zu verhindern. Die Primärprävention richtet sich an Risikogruppen, Gesunde und Personen ohne Krankheitssymptome.

Von der Primärprävention kann noch die primordiale Prävention abgegrenzt werden, die noch früher einsetzt. Bei ihr geht es darum, bereits dem Auftreten von Risikofaktoren vorzubeugen.

Sekundärprävention setzt beim Frühstadium einer Krankheit an. Sie dient der Früherkennung von Krankheiten und der Eindämmung ihres Fortschreitens (Progredienz) oder der Chronifizierung der Erkrankung. Oft ohne eine für die Betroffenen wahrnehmbare Krankheitssymptomatik hat der pathogenetische Prozess hier bereits seinen Anfang genommen. Zielgruppe sind Personen, die zwar als Gesunde oder Symptomlose an der Präventionsmaßnahme teilnehmen, durch die diagnostische Maßnahme aber zu Patienten werden.

Tertiärprävention findet nach einer Akutbehandlung oder der Manifestation einer Erkrankung statt. Mit ihr sollen Folgeschäden und Rückfälle verhindert werden. Sie richtet sich an Patienten mit chronischen Beeinträchtigungen und an Rehabilitanden. Ein Beispiel ist hier die Verhinderung von Rezidiven bei Tumorerkrankungen.

Weiterhin gibt es noch die Quartäre Prävention, die die Verhinderung unnötiger Medizin oder Verhinderung von Überdosierungen zum Ziel hat und das Prinzip des «primum non nocere» als einen Grundpfeiler aller Medizin berücksichtigt.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

Im Einklang mit der vorangehenden Beschreibung werden die folgenden Ausführungsformen offenbart, die alleine oder in jeglicher Kombination mit dem vorgenannten Ausführungsformen Teil der Erfindung sind.

### BEISPIELE

### Beispiel 1. pH-induziertes NO-Herstellungsverfahren

### 1.1 Material:

- Gerät zur Quantifizierung von NO: Eco physics CLD 822
- Reaktionskammer: Quarzglas, ca. 100x100x10mm (ca. 100ml Volumen)
- Pufferlösung: 150 mM Essigsäure, 150 mM NaOH in Aqua-dest
- Base: 1M NaOH
- Natrium-L-Ascorbat
- 1M NaNO₂

### 1.2 Versuchsführung

0,56 g Natrium-L-ascorbat wurden in 98,6 ml Pufferlösung gelöst, in die Reaktionskammer überführt und 1,4 ml NaNO₂ (1M) zugegeben. Die Natriumnitritkonzentration betrug demnach 14 mM, die Ascorbatkonzentration 28,3 mM. Für die Endlösung wurde ein pH-Wert von 5,0 gemessen.

Über einen Zeitraum von 60 min wurde in Abständen von jeweils 2-3 Minuten eine 200 µl Probe entnommen und der NO Gehalt mit Hilfe des CLD Systems quantifiziert.

### 1.3 Ergebnisse

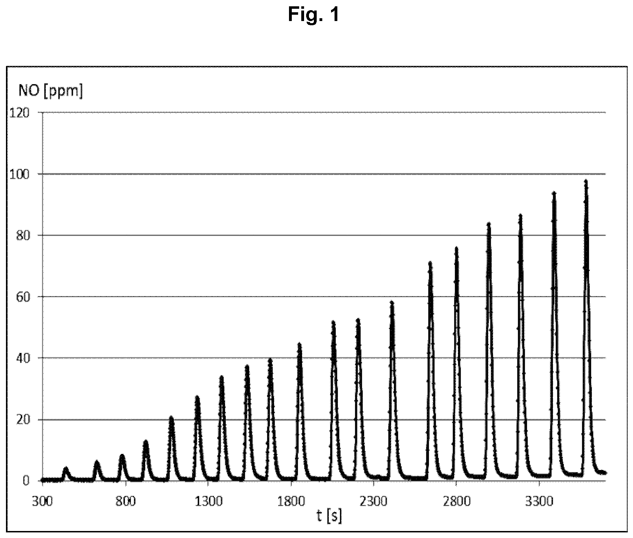

Die Ergebnisse der NO-Messungen in Abhängigkeit von der Reaktionszeit sind in Figur 1 dargestellt. Es ist ein kontinuierlicher Anstieg der NO-Konzentration zu beobachten, wobei nach 60 Minuten ein Wert erreicht wird, der einer Konzentration von 1,11 mM in der Flüssigkeit entspricht.

### FIGURENLEGENDEN

Die Erfindung wird nachfolgend anhand der Figuren näher erläutert ohne die Erfindung auf dieses zu beschränken. Es zeigen:
- Fig. 1:: Bei pH 5,0 und geeignetem Puffersystem wird nach ca. 30 min im Konzentrat (A) eine NO Konzentration von 1 mM erreicht. Verdünnung mit Aqua dest. (z.B. 1:10) führt zu einem erhöhten pH-Wert (z.B. 6,0) mit stabiler NO Konzentration (z. B. 100 µM).

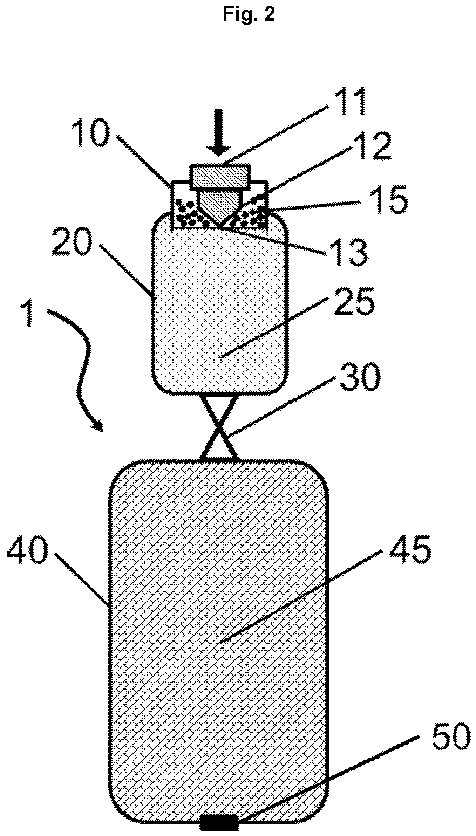

- Fig. 2:: Eine schematische Darstellung einer Vorrichtung 1 zur Durchführung des Dilutionsverfahrens mit einem ersten Behälter 10, der die erste Reaktionskomponente 15 in fester Form enthält (bevorzugt Natriumnitrit und Natriumascorbat). Diese erste Reaktionskomponente 15 kann durch einen Druck (schwarzer Pfeil) auf den Druckknopf 11 und Durchstechen der Trennmembran 13 mit dem Durchstechelement 12 zur Mischung mit der zweiten Reaktionskomponente 25, die im zweiten Behälter 20 vorgehalten wird, gebracht werden. Der zweite Behälter 20 ist über ein Ventil 30 mit dem dritten Behälter 40 flüssigkeitsführend verbunden. Nach Öffnen des Ventils 30 kann die azide NO-haltige Lösung im Behälter 20 durch die Verdünnungslösung als dritte Reaktionskomponente 45 verdünnt und gleichzeitig im pH-Wert angehoben werden.

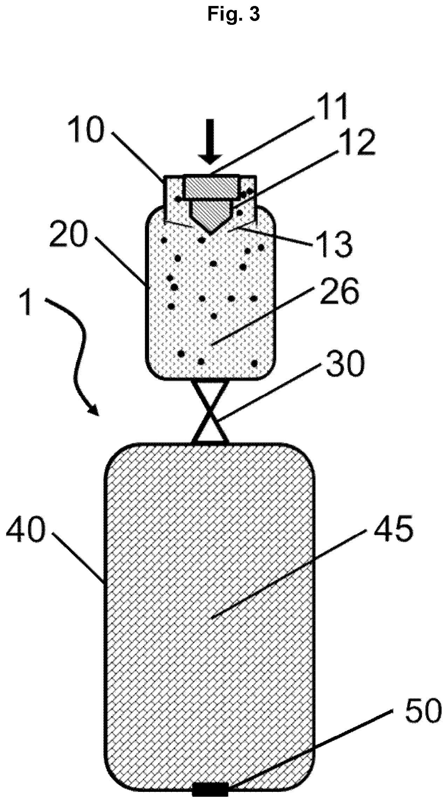

- Fig. 3:: Eine schematische Darstellung einer Vorrichtung 1 zur Durchführung des Dilutionsverfahrens gemäß Figur 2, wobei das Ergebnis der Druckknopfbetätigung dargestellt ist. Der eingedrückte Druckknopf 11 hat mit dem Durchstechelement 12 die Trennmembran 13 durchstochen und damit den ersten Behälter 10 und den zweiten Behälter 20 so miteinander verbunden, dass nun in ihnen ein Gemisch 26 aus der ersten und zweiten Reaktionskomponente erzielt worden ist.

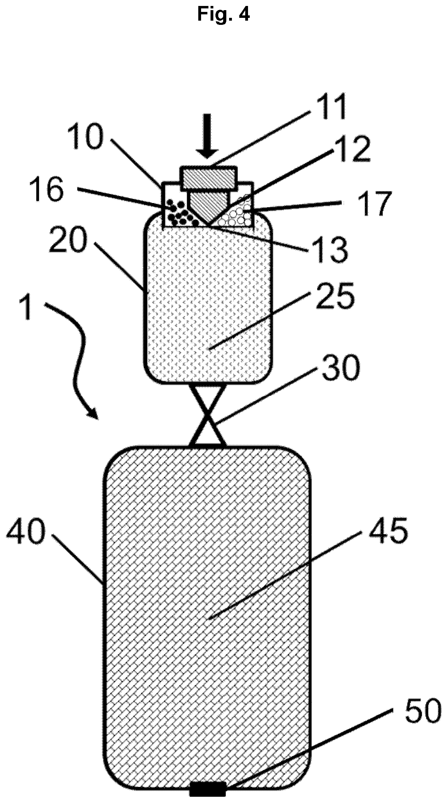

- Fig. 4:: Eine schematische Darstellung einer Vorrichtung 1 zur Durchführung des Dilutionsverfahrens gemäß Figur 2, wobei Nitritpartikel 16 als pH-labiler NO-Donor und Ascorbatpartikel 17 als Antioxidans in dem ersten Behälter 10 in zwei voneinander getrennten Kompartimenten vorliegen.

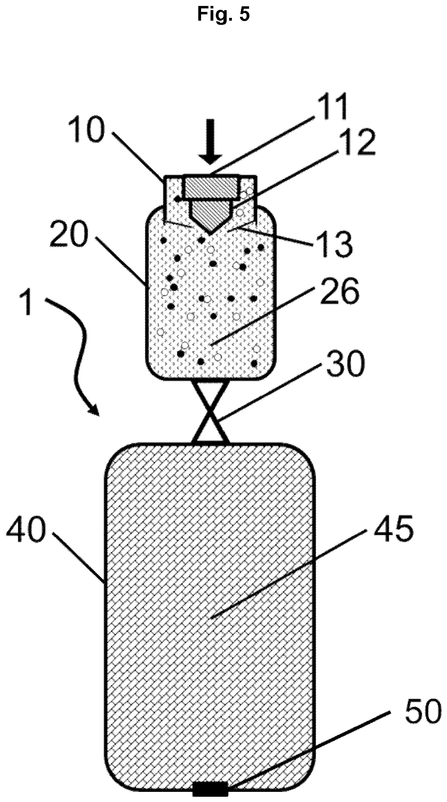

- Fig. 5:: Eine schematische Darstellung einer Vorrichtung 1 zur Durchführung des Dilutionsverfahrens gemäß Figur 4, wobei das Ergebnis der Druckknopfbetätigung dargestellt ist. Der eingedrückte Druckknopf 11 hat mit dem Durchstechelement 12 die Trennmembran 13 durchstochen und damit den ersten Behälter 10 und den zweiten Behälter 20 so miteinander verbunden, dass nun in ihnen ein Gemisch 26 aus den Nitritpartikeln und den Ascorbatpartikeln mit der flüssigen zweiten Reaktionskomponente erzielt worden ist.

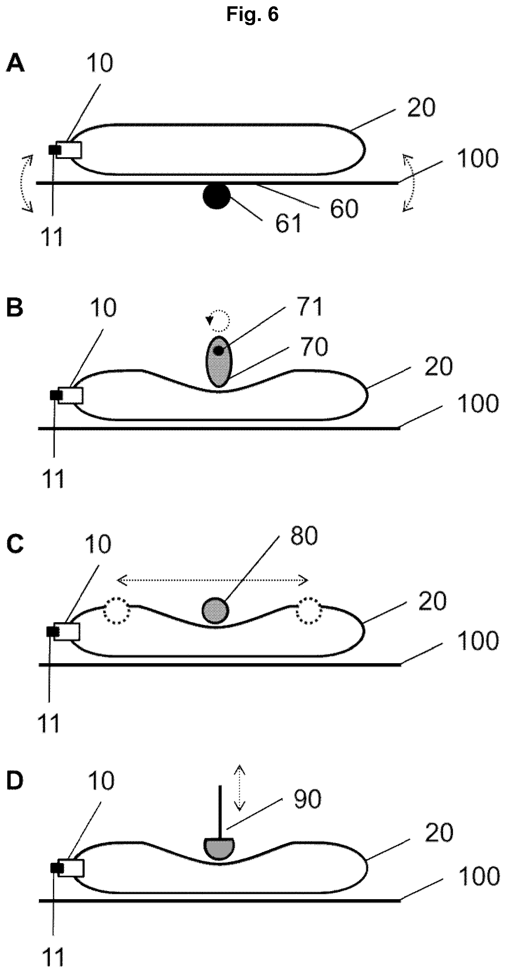

- Fig. 6: in A bis D vier verschiedene Mischvorrichtungen für den als Beutel ausgestalten zweiten Behälter 20 mit dem vorbefüllten Druckknopf 10,11 als ersten Behälter mit einer als Wipp-Platte 60 und Drehachse 61 ausgestalteten Trägerplatte 100 in A; mit einer Exzentervorrichtung 70 und einer Drehachse 71 in B; mit einer seitlich beweglichen Rolle 80 in C und mit einem auf- und abwärts beweglichen Stempel in D.

### BEZUGSZEICHEN

- 1: Vorrichtung zur Durchführung des Dilutionsverfahrens
- 10: Erster Behälter
- 11: Druckknopf
- 12: Durchstechelement
- 13: Trennmembran
- 15: Erste Reaktionskomponente
- 16: Nitritpartikel als erster Bestandteil der ersten Reaktionskomponente
- 17: Ascorbatpartikel als zweiter Bestandteil der ersten Reaktionskomponente
- 20: Zweiter Behälter
- 25: Zweite Reaktionskomponente
- 26: Gemisch aus erster und zweiter Reaktionskomponente
- 30: Ventil
- 40: Dritter Behälter
- 45: Dritte Reaktionskomponente
- 50: Anschluss für Entnahme von Flüssigkeit aus dem dritten Behälter
- 60: Wipp-Platte ( Wippbewegung siehe Pfeile)
- 61: Drehachse für Trägerplatte
- 70: Exzenterelement
- 71: Drehachse für Exzenterelement
- 80: seitlich bewegliche Rolle (gestrichelte Rollen = Extrempositionen)
- 90: Stempel
- 100: Trägerplatte für Beutel

## Patentansprüche

1. Verfahren zur Herstellung einer wässrigen Stickstoffmonoxid (NO)-haltigen Lösung umfassend die folgenden Schritte:
(a) Bereitstellen einer ersten Reaktionskomponente umfassend einen pH-labilen NO-Donor;
(b) Bereitstellen einer zweiten Reaktionskomponente, die ein wässrige Lösung mit einem pH-Wert zwischen 2,5 und 7,0 ist;
(c) Vermischen der ersten und zweiten Reaktionskomponente unter Bildung einer wässrigen Lösung mit einem pH-Wert zwischen 3,0 und 5,5 und Generierung von NO zur Ausbildung einer NO-haltigen wässrigen Lösung;
(d) Verdünnen der NO-haltigen wässrigen Lösung aus Schritt (c) durch Vermischen mit einer wässrigen Verdünnungslösung, so dass ein Verdünnungsfaktor von 5 bis 20 resultiert, und die wässrige Verdünnungslösung eine gepufferte Lösung oder destilliertes Wasser ist, so dass der pH-Wert der Mischung aus Schritt (c) um mindestens eine pH-Wertstufe erhöht wird.

2. Verfahren gemäß Anspruch 1, **dadurch gekennzeichnet, dass** die erste Reaktionskomponente als Feststoff, Schaum, Creme, Gel oder Flüssigkeit und bevorzugt als pulverförmiger oder granulärer Feststoff vorliegt.

3. Verfahren gemäß Anspruch 1 oder 2, **dadurch gekennzeichnet, dass** der pH-labile NO-Donor ausgewählt ist aus der Gruppe enthaltend anorganisches Nitritsalz, Alkylnitrite wie Isopentylnitrit, Diazeniumdiolat-Derivate, trans[RuCl([15]aneN4)NO]²⁺, 6-Nitrobenzo[a]pyrol, S-Nitroso-Glutathion, S-Nitroso-Thiol, S-Nitroso-N-Acetyl-D-Penicillamin (SNAP), Nitroanilin-Derivate, 2-Methyl-2-Nitrosopropan, Imidazoyl-Derivate, Nitratester, Hydroxylnitrosamin, Hydroxylamin, Hydroxyharnstoff, Natrium-Nitroprussid, und bevorzugt ein anorganisches Nitritsalz ist.

4. Verfahren gemäß Anspruch 1 bis 3, **dadurch gekennzeichnet, dass** der pH-labile NO-Donor ausgewählt ist aus der Gruppe LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, oder Ra(NO₂)₂ und Kombinationen hiervon und bevorzugt NaNO₂ ist.

5. Verfahren gemäß einem der vorangehenden Ansprüche, **dadurch gekennzeichnet, dass** das die erste Reaktionskomponente und/oder die zweite Reaktionskomponente und/oder die Verdünnungslösung mindestens ein Antioxidans enthält, und bevorzugt nur die erste Reaktionskomponente das mindestens eine Antioxidans enthält.

6. Verfahren gemäß einem der vorangehenden Ansprüche, **dadurch gekennzeichnet, dass** das mindestens eine Antioxidans ausgewählt ist aus der Gruppe enthaltend Ascorbat und Derivate hiervon, Tocopherol, -trienol, -monoenol und Derivate hiervon, Butylhydroxyanisol (BHA), Butylhydroxytoluol (BHT), Glutathion, Cystein, Thiomilchsäure, alpha-Liponsäure, p-Cumarsäure, Ferulasäure, Sinapinsäure, Kaffeesäure, Gallussäure, Procatechusäure, Syringasäure, Vanillinsäure, polyphenolische Verbindungen aus der Gruppe der Anthocyane, Flavonoide oder Phytoöstrogene, und bevorzugt ein Gemisch aus einem Ascorbinsäure-Derivat und einem Tocopherol-Derivat ist.

7. Verfahren gemäß einem der vorangehenden Ansprüche, **dadurch gekennzeichnet, dass** der Verfahrensschritt (c) eine Zeitdauer von zwischen einer 1 Minute und 60 Minuten, bevorzugt von zwischen 15 Minuten und 45 Minuten und besonders bevorzugt von 20 bis 30 Minuten aufweist.

8. Verfahren gemäß einem der vorangehenden Ansprüche, **dadurch gekennzeichnet, dass** die im Schritt (d) durch das Verdünnen resultierende wässrige Lösung eine NO-Konzentration von zwischen 10 µM und 1000 µM, bevorzugt von zwischen 20 µM und 500 µM und besonders bevorzugt von zwischen 75 und 200 µM aufweist.

9. Vorrichtung zur Durchführung eines Verfahrens gemäß einem der Ansprüche 1 bis 8, umfassend mindestens drei Behälter:
(a) ein erster Behälter zur Aufnahme der ersten Reaktionskomponente, der über eine Öffnung oder Leitung mit einem zweiten Behälter verbunden ist,
(b) ein zweiter Behälter zur Aufnahme der flüssigen zweiten Reaktionskomponente, wobei dieser Behälter darüber hinaus mit einer weiteren Öffnung oder Leitung zum Durchlass von Flüssigkeit mit einem dritten Behälter verbunden ist;
(c) ein dritter Behälter zur Aufnahme der Verdünnungsflüssigkeit;
wobei die Leitung oder Öffnung zwischen den einzelnen Behältern durch ein Absperrelement verschlossen ist, dass sich öffnen oder entfernen lässt, um die Verbindung zwischen den einzelnen Behältern herzustellen,
und wobei der erste Behälter ein mit der ersten Reaktionskomponente vorbefüllter Verschlussdeckel ist, der den zweiten Behälter abschließt und einen Auslösungsmechanismus aufweist, der bei Betätigung eine Vermischung der ersten Reaktionskomponente im Verschlussdeckel und der zweiten Reaktionskomponente im zweiten Behälter erlaubt.

10. Instillationseinheit umfassend eine Vorrichtung gemäß Anspruch 9, **dadurch gekennzeichnet, dass** sie ausgewählt ist aus der Gruppe aufweisend Wundauflage, Wundpflaster, Spray, Inhalator, Badevorrichtung und Duschvorrichtung, wobei die Instillationseinheit bevorzugt zur Unterdrucktherapie verwendet wird.

11. Instillationseinheit gemäß Anspruch 10 **dadurch gekennzeichnet, dass** sie ferner umfasst:
(a) Steuerungssystem zum Öffnen und Schließen der Absperrelemente zwischen den Vorrichtungsbehältern;
(b) optional ein Kontrollsystem zur Kontrolle des Öffnungsstatus der Absperrelemente;
(c) Pumpvorrichtung zum Transport oder Umwälzen der wässrigen Lösungen;
(d) optional ein Rührsystem zum Durchmischen der Flüssigkeiten in einem oder mehreren der Behälter der Vorrichtung;
(e) (optional) eine Temperiereinheit zur Temperierung der Reaktionskomponenten und oder der Verdünnungslösung und/oder der verdünnten NO-haltigen Lösung;
(f) eine Rechen- und Speichereinheit zur Steuerung der Komponenten der Instillationseinheit.

12. Vorrichtung gemäß Anspruch 9 oder Instillationseinheit gemäß Anspruch 10 oder 11 zur Verwendung bei der Behandlung oder Prävention von Erkrankungen, **dadurch gekennzeichnet, dass** die Körperoberfläche oder Hohlorgane des Patienten dem aus der Vorrichtung oder der Instillationseinheit freigesetzten NO-haltigen Flüssigkeit ausgesetzt wird.

13. Kosmetisches Verfahren umfassend die Einwirkung von NO-haltiger Flüssigkeit auf die Haut eines Menschen, **dadurch gekennzeichnet, dass** ein Verfahren gemäß Anspruch 1 bis 8, eine Vorrichtung gemäß Anspruch 9 oder eine Instillationseinheit gemäß Anspruch 10 oder 11 verwendet wird, wobei die Vorrichtung oder die Installationseinheit zur Herstellung der im kosmetischen Verfahren verwendeten NO-haltigen Flüssigkeit verwendet wird.

## Claims

1. Method for preparing an aqueous solution containing nitrogen monoxide (NO), comprising the following steps:
(a) providing a first reaction component comprising a pH-labile NO donor;
(b) providing a second reaction component which is an aqueous solution having a pH between 2.5 and 7.0;
(c) mixing the first and the second reaction component to form an aqueous solution having a pH between 3.0 and 5.5 and generating NO to form an aqueous solution containing NO;
(d) diluting the aqueous solution containing NO from step (c) by mixing with an aqueous dilution solution to result in a dilution factor of 5 to 20, and the aqueous dilution solution is a buffered solution or distilled water, so that the pH of the mixture from step (c) is increased by at least one pH step.

2. Method according to claim 1, **characterized in that** the first reaction component is in the form of a solid, foam, cream, gel or liquid and preferably as a powdered or granular solid.

3. Method according to claim 1 or 2, **characterized in that** the pH-labile NO donor is selected from the group comprising inorganic nitrite salt, alkyl nitrites such as isopentyl nitrite, diazeniumdiolate derivatives, trans[RuCl([15]aneN4)NO]²⁺, 6-nitrobenzo[a]pyrrole, S-nitrosoglutathione, S-nitrosothiol, S-nitroso-N-acetyl-D-penicillamine (SNAP), nitroaniline derivatives, 2-methyl-2-nitrosopropane, imidazoyl derivatives, nitrate esters, hydroxyl-nitrosamine, hydroxylamine, hydroxyurea, sodium nitroprusside, and is preferably an inorganic nitrite salt.

4. Method according to claims 1 to 3, **characterized in that** the pH-labile NO donor is selected from the group LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, or Ra(NO₂)₂ and combinations thereof and is preferably NaNO₂.

5. Method according to any of the preceding claims, **characterized in that** that the first reaction component and/or the second reaction component and/or the dilution solution contains at least one antioxidant, and preferably only the first reaction component contains the at least one antioxidant.

6. Method according to any of the preceding claims, **characterized in that** the at least one antioxidant is selected from the group comprising ascorbate and derivatives thereof, tocopherol, tocotrienol, tocomonoenol and derivatives thereof, butylhydroxyanisole (BHA), butylhydroxytoluene (BHT), glutathione, cysteine, thiolactic acid, alpha-lipoic acid, p-coumaric acid, ferulic acid, sinapic acid, caffeic acid, gallic acid, procatechuic acid, syringic acid, vanillic acid, polyphenolic compounds from the group of anthocyanins, flavonoids or phytoestrogens, and is preferably a mixture of an ascorbic acid derivative and a tocopherol derivative.

7. Method according to any of the preceding claims, **characterized in that** method step (c) has a duration of between 1 minute and 60 minutes, preferably between 15 minutes and 45 minutes and particularly preferably between 20 and 30 minutes.

8. Method according to any of the preceding claims, **characterized in that** the aqueous solution resulting from the dilution in step (d) has a NO concentration of between 10 µM and 1000 µM, preferably between 20 µM and 500 µM and particularly preferably between 75 and 200 µM.

9. Device for carrying out a method according to any of claims 1 to 8, comprising at least three containers:
(a) a first container for receiving the first reaction component, which is connected to a second container via an opening or line,
(b) a second container for receiving the liquid second reaction component, wherein said container is further connected to a third container by a further opening or line for the passage of liquid;
(c) a third container for receiving the dilution liquid;
wherein the line or opening between the individual containers is closed by a shut-off element that can be opened or removed to establish connection between the individual containers, and wherein the first container is a closure cap pre-filled with the first reaction component, which closes off the second container and has a trigger mechanism which, when actuated, allows mixing of the first reaction component in the closure cap and the second reaction component in the second container.

10. Instillation unit comprising a device according to claim 9, **characterized in that** it is selected from the group comprising wound dressing, wound plaster, spray, inhaler, bathing device and shower device, wherein the instillation unit is preferably used for negative pressure therapy.

11. Instillation unit according to claim 10, **characterized in that** it also comprises:
(a) control system for opening and closing the shut-off elements between the device containers;
(b) optionally a monitoring system for monitoring the opening status of the shut-off elements;
(c) pumping device for transporting or circulating the aqueous solutions;
(d) optionally a stirring system for mixing the liquids in one or more of the containers of the device;
(e) (optionally) a temperature control unit for controlling the temperature of the reaction components and/or the dilution solution and/or the diluted solution containing NO;
(f) a computing and storage unit for controlling the components of the instillation unit.

12. Device according to claim 9 or instillation unit according to claim 10 or 11 for use in the treatment or prevention of diseases, **characterized in that** the patient's body surface or hollow organs are exposed to the liquid containing NO released from the device or instillation unit.

13. Cosmetic procedure comprising the action of liquid containing NO on the skin of a human, **characterized in that** a method according to claims 1 to 8, a device according to claim 9 or an instillation unit according to claim 10 or 11 is used, the device or the installation unit being used for producing the liquid containing NO used in the cosmetic procedure.

## Revendications

1. Procédé permettant la préparation d'une solution aqueuse contenant du monoxyde d'azote (NO), comprenant les étapes suivantes :
(a) fourniture d'un premier composant de réaction comprenant un donneur de NO, labile par rapport au pH ;
(b) fourniture d'un second composant de réaction qui est une solution aqueuse avec un pH compris entre 2,5 et 7,0 ;
(c) mélange du premier et du second composant de réaction en formant une solution aqueuse avec un pH compris entre 3,0 et 5,5 et génération du NO permettant de réaliser une solution aqueuse contenant du NO ;
(d) dilution de la solution aqueuse contenant du NO de l'étape (c) en la mélangeant avec une solution de dilution aqueuse de sorte qu'un facteur de dilution allant de 5 à 20 en résulte, et la solution de dilution aqueuse est une solution tamponnée ou de l'eau distillée, de sorte que le pH du mélange de l'étape (c) est augmenté d'au moins un échelon de valeur de pH.

2. Procédé selon la revendication 1, **caractérisé en ce que** le premier composant de réaction se présente sous forme de solide, mousse, crème, gel ou liquide et de préférence sous forme de solide pulvérulent ou granulaire.

3. Procédé selon la revendication 1 ou 2, **caractérisé en ce que** le donneur de NO, labile par rapport au pH, est choisi dans le groupe contenant un sel de nitrite inorganique, des nitrites d'alkyle tels que le nitrite d'isopentyle, des dérivés de diolate de diazénium, le trans[RuCl([15]aneN4)NO]²⁺, le 6-nitrobenzo[a]pyrol, le S-nitroso-glutathion, le S-nitroso-thiol, le S-nitroso-N-acétyl-D-pénicillamine (SNAP), des dérivés de nitroaniline, le 2-méthyl-2-nitrosopropane, des dérivés d'imidazoyle, des esters de nitrate, l'hydroxylnitrosamine, l'hydroxylamine, l'hydroxyurée, le nitroprussiate de sodium, et est de préférence un sel inorganique de nitrite.

4. Procédé selon les revendications 1 à 3, **caractérisé en ce que** le donneur de NO, labile par rapport au pH, est choisi dans le groupe LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, ou Ra(NO₂)₂ et des combinaisons de ceux-ci et est de préférence NaNO₂.

5. Procédé selon l'une des revendications précédentes,
**caractérisé en ce que** le premier composant de réaction et/ou le second composant de réaction et/ou la solution de dilution contiennent au moins un antioxydant, et de préférence seul le premier composant de réaction contient l'au moins un antioxydant.

6. Procédé selon l'une des revendications précédentes,
**caractérisé en ce que** l'au moins un antioxydant est choisi dans le groupe contenant l'ascorbate et ses dérivés, le tocophérol, le tocotriénol, le tocomonoénol et leurs dérivés, le butylhydroxyanisole (BHA), le butylhydroxytoluène (BHT), le glutathion, la cystéine, l'acide thiolactique, l'acide alpha-lipoïque, l'acide p-coumarique, l'acide férulique, l'acide sinapique, l'acide caféique, l'acide gallique, l'acide procatéchique, l'acide syringique, l'acide vanillique, des composés polyphénoliques du groupe des anthocyanes, des flavonoïdes ou des phytoestrogènes, et est de préférence un mélange d'un dérivé d'acide ascorbique et d'un dérivé de tocophérol.

7. Procédé selon l'une des revendications précédentes,
**caractérisé en ce que** l'étape (c) de procédé présente une durée comprise entre 1 minute et 60 minutes, de préférence entre 15 minutes et 45 minutes, et de manière particulièrement préférée de 20 à 30 minutes.

8. Procédé selon l'une des revendications précédentes,
**caractérisé en ce que** la solution aqueuse résultant de la dilution à l'étape (d) présente une concentration en NO comprise entre 10 µM et 1000 µM, de préférence entre 20 µM et 500 µM et de manière particulièrement préférée entre 75 et 200 µM.

9. Dispositif permettant de réaliser un procédé selon l'une des revendications 1 à 8, comprenant au moins trois récipients :
(a) un premier récipient permettant la réception du premier composant de réaction et raccordé à un second récipient par l'intermédiaire d'une ouverture ou conduite,
(b) un second récipient permettant la réception du second composant de réaction liquide, dans lequel ledit récipient est en outre raccordé à un troisième récipient par une autre ouverture ou conduite pour le passage de liquide ;
(c) un troisième récipient permettant la réception du liquide de dilution ;
dans lequel la conduite ou l'ouverture entre les récipients individuels est fermé par un élément de verrouillage qui peut être ouvert ou retiré afin d'établir le raccordement entre les récipients individuels, et dans lequel le premier récipient est un couvercle de fermeture prérempli avec le premier composant de réaction, lequel ferme le second récipient et présente un mécanisme de déclenchement qui, lorsqu'il est actionné, permet un mélange du premier composant de réaction dans le couvercle de fermeture et du second composant de réaction dans le second récipient.

10. Unité d'instillation comprenant un dispositif selon la revendication 9, **caractérisée en ce qu'**elle est choisie dans le groupe constitué d'un revêtement pour lésion cutanée, pansement pour lésion cutanée, spray, inhalateur, dispositif formant baignoire et dispositif formant douche, dans laquelle l'unité d'instillation est utilisée de préférence pour une thérapie par pression négative.

11. Unité d'instillation selon la revendication 10, **caractérisée en ce qu'**elle comprend en outre :
(a) un système de commande pour l'ouverture et la fermeture des éléments de verrouillage entre les récipients de dispositif ;
(b) éventuellement, un système de contrôle pour le contrôle de l'état d'ouverture des éléments de verrouillage ;
(c) un dispositif de pompage pour le transport ou la circulation des solutions aqueuses ;
(d) éventuellement, un système d'agitation pour le mélange des liquides dans un ou plusieurs des récipients du dispositif ;
(e) (éventuellement) une unité d'équilibrage de température pour l'équilibrage de température des composants de réaction et/ou de la solution de dilution et/ou de la solution diluée contenant du NO ;
(f) une unité de calcul et de stockage pour la commande des composants de l'unité d'instillation.

12. Dispositif selon la revendication 9 ou unité d'instillation selon la revendication 10 ou 11, destiné à être utilisé dans le traitement ou la prévention de maladies, **caractérisé en ce que** la surface corporelle ou les organes creux du patient sont exposés au liquide contenant du NO libéré par le dispositif ou par l'unité d'instillation.

13. Procédé cosmétique comprenant l'action d'un liquide contenant du NO sur la peau d'un être humain, **caractérisé en ce qu'**un procédé selon les revendications 1 à 8, un dispositif selon la revendication 9 ou une unité d'instillation selon la revendication 10 ou 11 est utilisé, dans lequel le dispositif ou l'unité d'installation est utilisé pour préparer le liquide contenant du NO utilisé dans le procédé cosmétique.
